# EUROPEAN PATENT APPLICATION

(11) **EP 3 792 239 A1**
(43) Date of publication of application: **17.03.2021**
(21) Application number: 19197147.2
(22) Date of filing: 13.09.2019
(51) Int. Cl.: C07C 29/17, C07C 33/03

(54) **SELECTIVE HYDROGENATION**

(71) Applicant: DSM IP Assets B.V., 6411 TE Heerlen (NL)
(72) Inventor: ANDERSON, James, Aberdeen, Scotland AB24 2FX (GB); McCUE, Alan, Aberdeen, Scotland AB24 2FX (GB); MEDLOCK, Jonathan Alan, Aberdeen, Scotland AB24 2FX (GB); MITKIEWICZ, Kamila, Aberdeen, Scotland AB24 2FX (GB)
(74) Representative: Kurt, Manfred

(57) **Abstract**

The present invention is related to an improved selective hydrogenation of specific organic compounds, with hydrogen in the presence of a specific catalyst. The hydrogenated compounds obtained by the selective hydrogenation according to the present invention are important intermediates for the production of vitamins, carotenoids, perfume ingredients, and/or food or feed ingredients.

## Description

The present invention is related to an improved selective hydrogenation of specific organic compounds, which are defined in detail below, with hydrogen in the presence of a specific catalyst. The hydrogenated compounds obtained by the selective hydrogenation according to the present invention are important intermediates for the production of vitamins, carotenoids, perfume ingredients, and/or food or feed ingredients.

Selective catalytic hydrogenations of alkynols to alkenols are important processes in the fine chemicals industry. Pd-based catalysts are known to give the highest selectivity and yield. Preferential formation of olefinic alcohols is attributed to the stronger adsorption of acetylenic alcohols in comparison with the half-hydrogenation product. Catalytic performance of palladium is known to be strongly influenced by its dispersion, nature of support and the use of promoters and additives. Catalyst design taking into consideration these factors can allow a yield increase of target product and catalyst reuse.

Very common catalyst for such hydrogenations are very well known Lindlar type catalysts. In industry hydrogenations, which are usually carried out in stirred tank reactors with, the Lindlar catalyst (such as i.e. Pd/CaCO₃ modified by lead acetate) often requires the addition of an organic modifier (such as quinoline) to optimise the reaction results.

Therefore, the goal of the present invention was to find a reaction system, which allows to hydrogenate selectively specific alkynols without the disadvantages of commonly used catalyst.

Therefore, the present invention relates to a selective hydrogenation of a compound of formula (I) 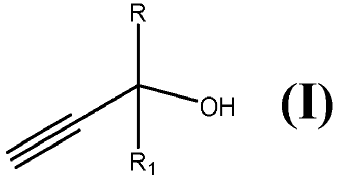 wherein
R is linear or branched C₁-C₃₅ alkyl or linear or branched C₃-C₃₅ alkenyl moiety, wherein the C chain can be substituted, and
R₁ is linear or branched C₁-C₄ alkyl, wherein the C chain can be substituted, with hydrogen in the presence of a catalyst comprising a Pd₄S phase.

The obtained reaction products of the hydrogenation are the compounds of formula (II) 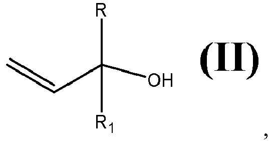 wherein the R and R₁ are as defined above.

The hydrogenation according to the present invention is usually and preferably carried out by using H₂ gas (pure or as a mixture with other gases).

In a preferred embodiment of the present invention compounds of formula (I), wherein R is a linear or branched C₁-C₃₀ alkyl moiety or a linear or branched C₅-C₃₀ alkenyl moiety, wherein the C chain can be substituted, and R₁ is a linear or branched C₁-C₄ alkyl moiety, wherein the C chain can be substituted, are used.

In a more preferred embodiment of the present invention compounds of formula (I), wherein R is a linear or branched C₁-C₁₆ alkyl moiety or a linear or branched C₆-C₁₆ alkenyl moiety, wherein the C chain can be substituted, and R₁ is a C₁-C₂ alkyl moiety, wherein the C chain can be substituted.

In a most preferred embodiment of the present invention compounds of formula (I), wherein R is a linear or branched CH₃, C₆-, C₁₁- or C₁₆- alkyl moiety or a linear or branched C₆-, C₁₁- or C₁₆- alkenyl moiety, and R₁ is a C₁-C₂ alkyl moiety.

Especially preferred compounds of formula (I) are the following:

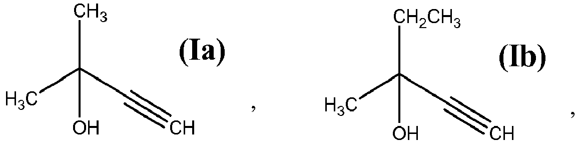

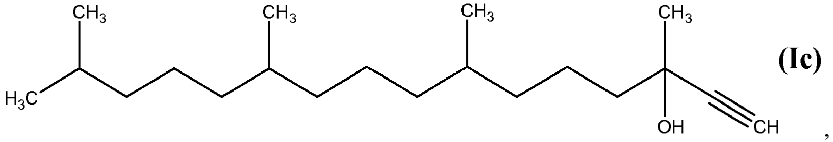

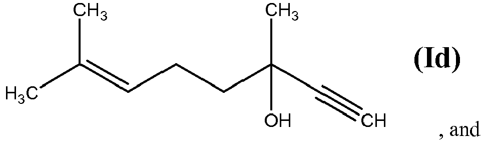

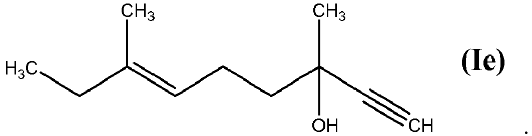

The catalyst which is used for the selective hydrogenation according to the present invention is a catalyst comprising a Pd₄S phase.

Such a catalyst is known from the prior art for other reactions. (i.e. D. Albani et al, Nature Communications, 2018(9), 1-11). For the alkyne hydrogenation described in the prior art catalysts comprising a Pd₄S phase are not recommended, because of poor performance.

The catalyst comprising a Pd₄S phase can be used with a support or without any support.

When using a support, the support can be any commonly used support, such as carbon or an inorganic carrier. Preferred inorganic carriers are oxides or carbonates. Preferred oxides are oxides of silicon, aluminium, zinc, titanium or cerium.

The catalyst can be prepared according to the methods disclosed and described in the prior art.

The selective hydrogenation according to the present invention can be carried out with or without any solvents.

When using a solvent, then the solvent is to be inert (to hydrogenations). Suitable solvents are hydrocarbons, halogenated hydrocarbons, alcohols, ethers, esters, carbonates, amides, nitriles and ketones and mixtures thereof.

The selective hydrogenation according to the present invention is usually and preferably carried out at elevated reaction temperatures.

Usually the selective hydrogenation is carried at a temperature above 40°C. preferably at a temperature between 40°C to 100°C.

The selective hydrogenation according to present invention is usually carried out at ambient to elevated pressure. Usually 1 bar to 10bar absolute.

An important advantage of the present invention is that the selective hydrogenation according to the present invention can be carried out without any organic modifier (such as quinoline).

The following examples serve to illustrate the invention. If not otherwise stated all parts are given are related to the weight and the temperature is given in °C

### Examples

### Preparation of unsupported catalysts

PdS powder (Alfa Aesar) was thermally activated in a flow of hydrogen gas (140 ml min⁻¹) at temperatures between 350-425 °C for 1 h. The obtained samples were denoted as PdS_bulk_350 °C or PdS_bulk_425 °C to reflect the reduction temperature.

### Preparation of supported catalysts

PdSO₄.H2O was dissolved in deionised water with the aid of sonication for 30 min, before being combined with support and stirred vigorously as water was evaporated at 90 °C. Thereafter, the samples were ground and subjected to thermal activation (140 ml min⁻¹ hydrogen gas at 250°C for 1 h).

### Hydrogenation reactions

Liquid phase hydrogenation reactions were performed at 1 bar and 60 °C in a batch reactor, stirring at 500 rpm, using 25 mg of catalyst and 50 ml of alkyne solution (0.1 mol L⁻¹) in heptane. Before introducing H2 (20 ml min⁻¹) the mixture was purged with N2 (40 ml min⁻¹) until desired temperature was reached. Reaction product distributions were determined by GC-FID (Bruker 430) equipped with Stabilwax capillary column.

### Results of hydrogenation reactions with unsupported catalysts

Hydrogenation of MBY (2-methyl-3-butyn-2-ol) to MBE (2-methyl-3-buten-2-ol). The reference Lindlar catalyst was obtained from Alfa Aesar and was used as received.

| Example | Catalyst | MBY Conv. at maximum MBE conc. | Maximum MBE Selectivity (%) | Maximum MBE yield (%) |
|---|---|---|---|---|
| Comparison | Lindlar | 94 | 79 | 74 |
| 1 | PdS bulk_350 °C | 91 | 93 | 84 |
| 2 | PdS bulk_425 °C | 91 | 86 | 78 |

### Results of hydrogenation reactions with supported catalysts

Hydrogenation of MBY (2-methyl-3-butyn-2-ol) to MBE (2-methyl-3-buten-2-ol). The reference Lindlar catalyst was obtained from Alfa Aesar and was used as received.

| Example | Catalyst | MBY Conv. at maximum MBE conc. | Maximum MBE Selectivity (%) | Maximum MBE yield (%) |
|---|---|---|---|---|
| Comparison | Lindlar | 94 | 79 | 74 |
| 3 | 1 wt% Pd on ZnO | 95 | 86 | 82 |
| 4 | 1 wt% Pd on Silica-alumina | 93 | 89 | 82 |
| 5 | 1 wt% Pd on Carbon nanofibre | 95 | 81 | 78 |

It can be seen that the catalyst according to the present invention is leading to better results that a Lindlar catalyst.

## Claims

1. Selective hydrogenation of a compound of formula (I) 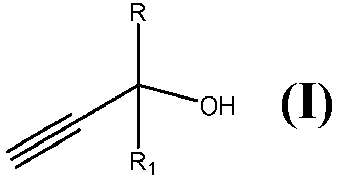 wherein
R is linear or branched C₁-C₃₅ alkyl or linear or branched C₃-C₃₅ alkenyl moiety, wherein the C chain can be substituted, and
R₁ is linear or branched C₁-C₄ alkyl, wherein the C chain can be substituted, with hydrogen in the presence of a Pd₄S catalyst.

2. The selective hydrogenation according to claim 1, wherein
R is a linear or branched C₁-C₃₀ alkyl moiety or a linear or branched C₅-C₃₀ alkenyl moiety, wherein the C chain can be substituted, and R₁ is a linear or branched C₁-C₄ alkyl moiety, wherein the C chain can be substituted.

3. The selective hydrogenation according to claim 1, wherein
R is a linear or branched C₁-C₁₆ alkyl moiety or a linear or branched C₆-C₁₆ alkenyl moiety, wherein the C chain can be substituted, and R₁ is a C₁-C₂ alkyl moiety, wherein the C chain can be substituted.

4. The selective hydrogenation according to claim 1, wherein
R is a linear or branched CH₃, C₆-, C₁₁- or C₁₆- alkyl moiety or a linear or branched C₆-, C₁₁- or C₁₆- alkenyl moiety, and R₁ is a C₁-C₂ alkyl moiety.

5. The selective hydrogenation according to claim 1, wherein compounds of formula (Ia), (Ib), (Ic). (Id) or (Ie) 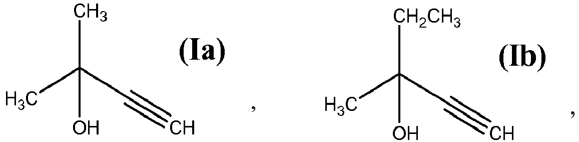 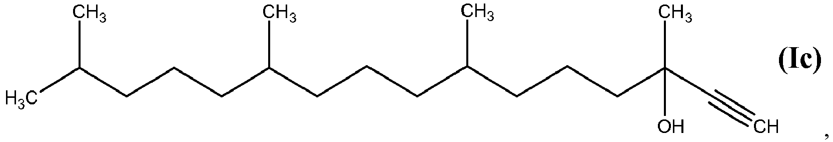 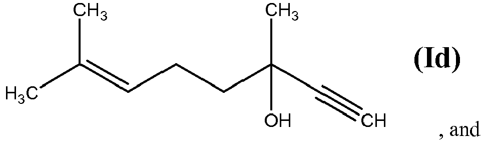 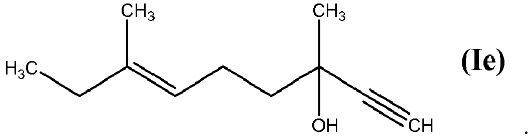 are used.

6. The selective hydrogenation according to any of the preceding claims, wherein by using H₂ gas (pure or as a mixture with other gases).

7. The selective hydrogenation according to any of the preceding claims, wherein the hydrogenation is carried out without any solvents.

8. The selective hydrogenation according to any of the preceding claims 1 - 6, wherein the hydrogenation is carried out in at least one solvent.

9. The selective hydrogenation according to claim 8, wherein the solvent is chosen from the group consisting of hydrocarbons, halogenated hydrocarbons, alcohols, ethers, esters, carbonates, amides, nitriles and ketones and mixtures thereof.

10. The selective hydrogenation according to any of the preceding claims, wherein the selective hydrogenation is carried out at elevated reaction temperatures.

11. The selective hydrogenation according to claim 10, wherein the temperature is between 40°C to 100°C.

12. The selective hydrogenation according to any of the preceding claims, wherein the selective hydrogenation is carried out at elevated pressure.

13. The selective hydrogenation according to claim 12, wherein the selective hydrogenation is carried out at 1 bar to 10bar.

14. The selective hydrogenation according to any of the preceding claims, wherein the selective hydrogenation is carried out without any organic modifier.
